(19) Europäisches Patentamt
European Patent Office
Office européen des brevets

(11) Veröffentlichungsnummer: **0 025 519**
**A1**

(12) # EUROPÄISCHE PATENTANMELDUNG

(21) Anmeldenummer: **80104895.0**

(22) Anmeldetag: **16.08.80**

(51) Int. Cl.³: **C 07 C 149/36,** C 07 C 148/00

(30) Priorität: **12.09.79 DE 2936803**

(43) Veröffentlichungstag der Anmeldung: **25.03.81**
**Patentblatt 81/12**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Wedemeyer, Karlfried, Dr., Bilharzstrasse 7, D-5000 Köln 80 (DE)**
Erfinder: **Fiege, Helmut, Dr., Walter-Flex-Strasse 8, D-5090 Leverkusen 1 (DE)**

(54) **Verfahren zur Herstellung von 2-Alkyl- bzw. 2-Arylthiomethylphenol.**

(57) Verfahren zur Herstellung von 2-Alkyl- oder 2-Arylthiomethylphenolen der Formel I

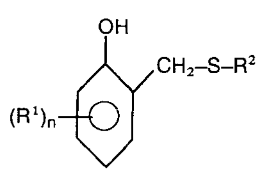

(I)

durch gleichzeitiges Umsetzen von Phenolen mit Formaldehyd oder einer Formaldehyd liefernden Verbindung mit einem Mercaptan oder Thiophenol, dadurch gekennzeichnet, daß man die Umsetzung bei Temperaturen von 90 bis 220°C in Gegenwart von Lithium-, Magnesium-, Calcium-, Strontium-, Barium-, Zink-, Cadmium-, Blei-, Mangan-, Kobalt-, Nickel-, Kupfer-, Eisen-, Chrom- und/oder Aluminium-Verbindungen als Katalysator durchführt.

EP 0 025 519 A1

0025519

BAYER AKTIENGESELLSCHAFT 5090 Leverkusen, Bayerwerk
Zentralbereich
Patente, Marken und Lizenzen Rt/ABc
Typ IVa/ZP

Verfahren zur Herstellung von 2-Alkyl- bzw. 2-Arylthiomethylphenol

Die Erfindung betrifft ein neues Verfahren zur Herstellung von 2-Alkyl- bzw. 2-Arylthiomethylphenolen.

Verbindungen dieses Typs dienen z.B. als Zwischenprodukte bei der Herstellung von Pflanzenschutzmitteln.

Es ist bekannt, daß man in 2,6- oder 2,4-Dialkylphenole durch Umsetzen mit Mercaptanen und Formaldehyd in Gegenwart von 0,1 - 1 Mol einer starken Base (wie z.B. Kaliumhydroxid) pro Mol Dialkylphenol eine Alkylthiomethylgruppe in die noch freie para- bzw. ortho-Stellung einführen kann. Bei Alkylphenolen mit freier ortho- und para-Position tritt die Alkylthiomethylgruppe dagegen gleichzeitig sowohl in die ortho- als auch in die para-Stellung in den Phenolkern ein [vgl. DAS 1 593 821, Deutsche Advance 1967]. Eine selektive Einführung der Alkylthiomethylgruppe in ortho-Stellung bei Phenolen mit noch freier para-Stellung durch Umsetzen mit Formaldehyd und einem Mercaptan wird nicht beschrieben.

Es ist ferner bekannt, daß man α-Aryl- oder α-Alkylthiomethylnaphthole durch Umsetzen z.B. von ß-Naphthol mit

Formaldehyd und Aryl- oder Alkylmercaptanen erhält (vgl. F. POPPELSDORF, S.J. HOLT, J. Chem. Soc. 1954, 1124 ff.). Die Reaktion läuft jedoch nur ab, wenn gleichzeitig erhebliche Mengen Triethylamin (0,5 Mol/Mol Phenol) als Hilfsbase anwesend sind, und auch dann erfordert sie zur Erzielung befriedigender Ausbeuten noch Reaktionszeiten von ca. 6 Tagen.

Überträgt man diese Reaktion auf Phenol, das bekanntlich drei reaktionsfähige Stellen besitzt, so entsteht ein Reaktionsgemisch, in dem neben dem gewünschten 2-Aryl- oder 2-Alkylthiomethylphenol noch erhebliche Mengen an isomeren 4-Aryl- oder 4-Alkylthiomethylphenol sowie Homologe und Harze enthalten sind. Dadurch wird nicht nur die Ausbeute an 2-Aryl- oder 2-Alkylthiomethylphenol stark vermindert, sondern auch seine Isolierung so erschwert, daß sie mit technisch vertretbarem Aufwand nicht mehr möglich ist.

Aus diesem Grund geht man bei den bekannten technischen Herstellungsverfahren für 2-Alkylthiomethylphenole von einem 2-Dimethylaminophenol (einer MANNICH-Base),

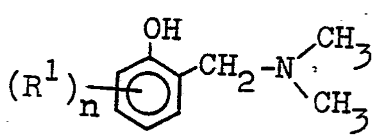

(A)

einem 2-Chlormethylphenol

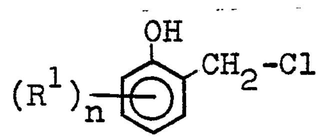

(B)

oder einem 2-Hydroxymethylphenol

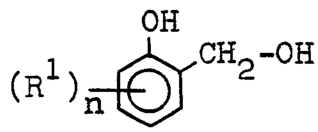

(C)

aus, das man mit entsprechenden Mercaptanen oder Mercaptane liefernden Verbindungen umsetzt [Lit. zur Umsetzung von Verbindung (A) s. z.B. DAS 2 614 875, BAYER AG; von (B): DT-PS 1 910 588, BAYER AG; JA 74 20 191; C.A. 82, 86 197 x; von (C): Le A 19 066]. Der Nachteil dieser Verfahren liegt vor allem darin, daß die Verbindungen A, B oder C nicht gut zugänglich sind und zuvor mit nicht unbeträchtlichem Aufwand an Zeit, Material bzw. technischen Hilfsmitteln hergestellt werden müssen.

Es war daher sehr wünschenswert, ein Verfahren zu finden, das die umständliche Herstellung dieser Vorstufen vermeidet und durch direkte Umsetzung der betreffenden Phenole mit Formaldehyd und einem Mercaptan die selektive Herstellung von 2-Alkyl- bzw. 2-Arylthiomethylphenolen des Typs I in einem Schritt gestattet. Ein solches Verfahren ist bisher noch nicht bekannt.

Es wurde nun gefunden, daß man mit hoher Selektivität und Ausbeute 2-Alkyl- oder 2-Arylthiomethylphenole der Formel I

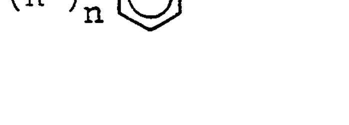

(I)

in der

n für 1, 2 oder 3 steht,

$R^1$ in 3-, 5- und/oder 6-Stellung zur Hydroxygruppe steht und entweder einzeln oder unabhängig voneinander für Wasserstoff, Alkyl, Cycloalkyl, Aryl, Aralkyl, Alkoxy und/oder Halogen bedeutet,

$R^2$ für gegebenenfalls substituierten Alkyl mit 1 bis 12 C-Atomen, für gegebenenfalls substituiertes Cycloalkyl mit 3 bis 6 C-Atomen, für gegebenenfalls substituiertes Phenyl oder gegebenenfalls substituiertes Aralkyl steht,

durch gleichzeitig Umsetzen von Phenolen der Formel

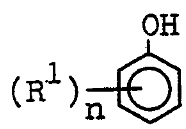

II

mit Formaldehyd oder einer Formaldehyd liefernden Verbindung mit einem Mercaptan oder Thiophenol der Formel

$$R^2\text{-S-H} \qquad \text{III}$$

erhält, wenn man die Umsetzung bei Temperaturen von 90 bis 220°C in Gegenwart von Lithium-, Magnesium-, Calcium-, Strontium-, Barium-, Zink-, Cadmium-, Blei-, Mangan-, Kobalt-, Nickel-, Kupfer-, Eisen-, Chrom-, und/oder Aluminium-Verbindungen als Katalysator durchführt.

Daß bei dem erfindungsgemäßen Verfahren 2-Alkylthiomethylphenole in guten Ausbeuten mit so hoher Selektivität gebildet werden, ist nach dem Stand der Technik als ausgesprochen überraschend zu bezeichnen:

Es ist bekannt, daß Metallkationen mit hoher Wertigkeit und/oder kleinem Ionenradius die Umsetzung von Phenol mit Formaldehyd zu 2-Hydroxymethylphenol begünstigen [s.D.A. FRASER u.a., J. appl. Chem. 7 (1957), 676 - 700]. Es war ferner bekannt, daß man 2-Hydroxymethylphenol mit Alkyl- oder Arylmercaptanen zu den entsprechenden 2-Alkyl- oder 2-Arylthiomethylphenolen umsetzen kann [s. die noch nicht zum Stand der Technik gehörende deutsche Anmeldung P 28 38 273.2 = Le A 19 066]. Führt man die Umsetzung von Phenol mit Formaldehyd in Gegenwart solcher Metallkationen jedoch bei den erfindungsgemäßen Temperaturen von über 90°C durch, so reagiert das 2-Hydroxymethylphenol sehr schnell und weitgehend mit sich selbst und dem noch vorhandenen Phenol zu Phenol-Formaldehyd-Harzen (sog. Novolaken) weiter [S. D.A. FRASER u.a., J. appl. Chem. 7 (1957), 676 - 700]. Das Vorherrschen solcher Verharzungsreaktionen war daher auch bei den erfindungsgemäßen Bedingungen zu erwarten, so daß es der Überwindung erheblicher Vorurteile bedurfte, die Reaktion bei so hohen Temperaturen durchzuführen.

Führt man die Umsetzung von Phenol mit Formaldehyd und dem Mercaptan in Gegenwart der erfindungsgemäßen Katalysatoren bei niedrigeren (als den erfindungsgemäßen), für die 2-Hydroxymethylphenol-Bildung günstigen Temperaturen durch, so stellt man nach Reaktionszeiten, die für die 2-Hydroxymethylphenol-Bildung sonst optimal sind,

fest, daß weder 2-Hydroxymethylphenol noch 2-Alkylthiomethylphenol in nennenswerter Ausbeute gebildet wurden. Mit anderen Worten heißt dies, daß die für die Phenol/Formaldehyd-Umsetzung bekannte Katalysatorwirkung bei gleichzeitiger Anwesenheit des Mercaptans praktisch verloren geht. Daß die Katalysatoren bei den erfindungsgemäßen Temperaturen wieder wirksam werden und die Umsetzung sogar - ohne wesentliche Verharzung selektiv zum 2-Alkylthiomethylphenol führt, war nach den vorausgegangenen Befunden nicht zu erwarten und sehr überraschend.

Das erfindungsgemäße Verfahren weist eine Reihe von Vorteilen auf, von denen hier nur einige genannt seien: Da es aus nur einer Stufe besteht, entfällt der bisher erforderliche beträchtliche Aufwand für die Herstellung einer definierten Zwischenverbindung. Die Reaktionsführung ist unproblematisch, und die Reaktionsparameter sind in weiten Grenzen frei wählbar. Die Ausbeuten sind hoch, und durch Wahl entsprechend hoher Temperaturen können kurze Reaktionszeiten und damit auch hohe Raum/Zeit-Ausbeuten erzielt werden. Die Anwendung eines entsprechenden Phenolüberschusses erlaubt auch bei Mercaptanen mit niedrigem Siedepunkt ein "druckloses" Arbeiten. Die Katalysatoren sind technisch gut zugänglich und brauchen nur in sehr geringen Mengen zugegeben zu werden. Das als Co-Produkt entstehende Wasser ist destillativ leicht abzutrennen. Die Einsatzmaterialien brauchen nicht wasserfrei zu sein. Besonders Korrosionsprobleme treten nicht auf.

Bei Verwendung von Ethylmercaptan, Formaldehyd und Phenol als Ausgangsverbindungen kann die Reaktion durch das folgende Formelschema wiedergegben werden:

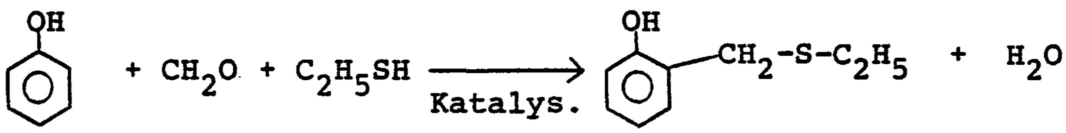


Die als Ausgangsstoff zu verwendenden Phenole sind durch die Formel (II) allgemein definiert. In dieser Formel stehen die Reste $R^1$ einzeln und unabhängig voneinander vorzugsweise für Wasserstoff, geradkettiges und/oder verzweigtes Alkyl mit 1 bis 6 C-Atomen, Cycloalkyl mit 3 bis 6 C-Atomen, Phenyl, Benzyl, $C_1$- bis $C_4$-Alkoxy und Halogen. Besonders bevorzugt als Ausgangsstoff (II) sind Phenole mit n = 1 und der vorgenannten Bedeutung für $R^1$. Ganz besonders bevorzugt als Ausgangsstoffe (II) ist Phenol selbst.

Phenole der Formel (II) sind bekannt oder können nach bekannten Verfahren hergestellt werden (s. Houben-Weyl, Bd. 6/1c, 4. Aufl. 1976).

Die erfindungsgemäß als Ausgangsstoffe zu verwendenden Mercaptane sind durch Formel (III) allgemein definiert. In dieser Formel steht der Rest $R^2$ vorzugsweise für geradkettiges oder verzweigtes Alkyl mit 1 bis 12 C-Atomen oder alkoxysubstituiertes Alkyl, Cycloalkyl mit 3 bis 6 C-Atomen, Phenyl, Benzyl, alkyl- und/oder halogensubstituiertes Phenyl.

Mercaptane der Formel (III) sind an sich bekannt oder können analog bekannter Verfahren hergestellt werden.

Der als Ausgangsstoff zu verwendende Formaldehyd kann in verschiedener Form eingesetzt werden, beispielsweise als Gas, gelöst in einem Lösungsmittel, in polymerer Form oder auch in Form von Formaldehyd liefernden Verbindungen (s. J.F. WALKER: "Formaldehyd", 3. Aufl. 1964, Reinhold Publishing Corp.). Bevorzugt verwendet werden wäßrige Formaldehyd-Lösungen (Formalin) oder Paraformaldehyd.

Man kann den Formaldehyd auch zusammen mit dem betreffenden Mercaptan in Form des bei der Umsetzung dieser beiden Verbindungen sich bildenden Thiomethanols (Halbmarcaptals) einsetzen. Zur Bildung solcher Thiomethanole s. T.G. LEVI, Gazz. chim. ital. 62 (1932) 775 - 780; H. BÖHME, H.P. TELZ Ann. Chem. 620 (1959) 1 - 4.

Bei der Durchführung des Verfahrens werden pro Mol Formaldehyd mindestens 0,8 Mol des betreffenden Phenols eingesetzt. Bei Phenolen mit zwei freien ortho-Positionen ist es besonders günstig, ein Phenol/Formaldehyd-Molverhältnis von über 0,8 anzuwenden, weil dadurch der Einführung einer zweiten Alkyl- bzw. Arylthiomethyl-Gruppe in die 6-Stellung des Phenols entgegengewirkt und geichzeitig eine reaktionstechnisch günstige Verdünnung des Reaktionsmedium erzielt wird. Dadurch sind dem Phenol/Formaldehyd-Verhältnis nach oben keine Grenzen gesetzt. Aus praktischen Gründen wird man gewöhnlich

bei Phenol/Formaldehyd-Verhältnissen zwischen 1 bis 20, insbesondere zwischen 1,5 bis 15 arbeiten.

Bei der Durchführung des erfindungsgemäßen Verfahrens kann die auf 1 Mol Formaldehyd einzusetzende Mercaptanmenge in weiten Grenzen schwanken. Gewöhnlich arbeitet man mit Mengen um 1 Mol Mercaptan pro Mol Formaldehyd. Da das Mercaptan gleichzeitig auch als Verdünnungsmittel dienen kann, sind auch dem Mercaptan/Formaldehyd-Verhältnis nach oben keine Grenzen gesetzt.

Bei dem erfindungsgemäßen Verfahren kann als Verdünnungsmittel außer dem betreffenden Phenol und dem betreffenden Mercaptan auch noch jedes beliebige andere inerte protische oder aprotische Lösungsmittel verwendet werden.

Von den erfindungsgemäß zu verwendenden Metallverbindungen haben sich besonders Lithium-, Magnesium- Calcium-, Strontium-, Barium-, Zink-, Cadmium-, Blei-, Mangan- und/oder Kobalt-Verbindungen bewährt.

Die Mengen, in denen die erfindungsgemäß zu verwendenden Katalysatoren eingesetzt werden, können in weiten Grenzen schwanken. Die Katalysatorwirkung macht sich bereits bei Zusätzen von $5.10^{-6}$ Mol Metallverbindung je Mol Phenol deutlich bemerkbar. Es können auch 0,1 Mol oder mehr Metallverbindung je Mol Phenol eingesetzt werden, jedoch bieten diese hohen Zusätze im allgemeinen keinen Vorteil. Im allgemeinen haben sich Zusätze von $1.10^{-5}$ bis $1.10^{-2}$ Mol, insbesondere $5.10^{-5}$ bis $5.10^{-3}$ Mol, Metallverbindung je Mol Phenol bewährt.

Die erfindungsgemäß als Katalysator einzusetzenden Metallverbindungen können in Form anorganischer oder organischer Verbindungen eingesetzt werden, beispielsweise in Form ihrer - gegebenenfalls hydratwasserhaltigen - Oxide, Oxidhydrate, Hydroxide, Carbonate, Nitrate, Sulfide, Sulfite, Sulfate, Phosphate, Borate, Plumbate, Chromate, Manganate, Formiate, Acetate. Propionate, Valerianate, Oxalate, Benzoate, Salicylate, Tartrate, Lactate, Zitrate, Alkoholate, Phenolate, Mercaptide oder beispielsweise auch als Alkyl- oder Arylverbindungen der betreffenden Metalle. Die Phenolate können gegebenenfalls auch vor der eigentlichen Umsetzung erst in situ aus dem betreffenden Metall und dem noch trocknenen Phenol hergestellt werden.

In den erfindungsgemäß einzusetzenden Metallverbindungen des Bleis, Mangans, Kobalts, Nickels, Eisens und/oder Chroms können diese Metalle in verschiedenen Wertigkeitsstufen vorliegen.

Die Wahl der einzusetzenden Metallverbindung erfolgt in erster Linie nach praktischen Gesichtspunkten, beispielsweise nach der Verfügbarkeit. Bevorzugt werden die Metallverbindungen in Form ihrer Oxide, Hydroxide, Carbonate, Formiate, Acetate oder Phenolate eingesetzt. Selbstverständlich können auch Kombinationen der erfindungsgemäßen Katalysatoren benutzt werden.

Die Metallverbindung kann als Feststoff, vorzugsweise in feinverteilter Form, oder in gelöster oder suspendierter Form den Reaktionskomponenten zugesetzt werden. Lösungs- oder Suspensionsmittel können Phenol, das betreffende Mercaptan oder beliebige andere inerte protische oder aprotische Stoffe sein, beispielsweise Wasser, Methanol, Ethanol, Propanol, Cyclohexan, Toluol, Chlorbenzol.

Die Metallverbindung kann beim erfindungsgemäßen Verfahren durchaus Veränderungen erfahren und braucht auch nicht vollständig im Reaktionsmedium löslich zu sein. Die Veränderung der eingesetzten Metallverbindung kann z.B. auch mit einem Wechsel in der Wertigkeit des Metalls verbunden sein.

Das erfindungsgemäße Verfahren wird üblicherweise so durchgeführt, daß man ein Gemisch aus Phenol, Formaldehyd bzw. Formaldehyd abgebender Verbindung, Mercaptan auf eine Temperatur von 90°C oder höher erhitzt und so lange bei $\geq$ 90°C beläßt, bis die Umsetzung zum 2-Alkyl- bzw. 2-Arylthiomethylphenol im gewünschten Umfang stattgefunden hat. Im allgemeinen wird man die Umsetzung bei Temperaturen zwischen 90 bis 220°C, vorzugsweise zwischen 100 und 200°C, durchführen.

Die erforderliche Reaktionszeit läßt sich analytisch leicht, z.B. über den maßanalytisch bestimmten Mercaptan-Umsatz oder über die durch Gaschromatographie oder Hochdruckflüssigkeitschromatographie bestimmte 2-Alkyl- bzw. 2-Arylthiomethylphenol-Bildung, verfolgen.

Die Umsetzung kann bei Normaldruck durchgeführt werden, beispielsweise auch unter Rückflüß, oder unter Eigendruck im Autoklaven. Die Arbeitsweise richtet sich nach dem Dampfdruck des Reaktionsgemisches, den technischen Gegebenheiten und der gewünschten Reaktionszeit. Das Arbeiten unter Eigendruck im Autoklaven ermöglicht höhere Reaktionstemperaturen und damit kürzere Reaktionszeiten. Die Arbeitsweise im Autoklaven kann bei niedrigsiedenden Mercaptanen zweckmäßig sein. Überschüssiges Phenol und/oder hochsiedende inerte Verdünnungsmittel ermöglichen ein Arbeiten bei höheren Temperaturen auch unter Normaldruck.

Die Vermischung von Katalysator, Phenol, Mercaptan und Formaldehyd bzw. Formaldehyd abgebender Komponente erfolgt gewöhnlich bei einer Temperatur, die unter dem erfindungsgemäß beanspruchten Temperaturbereich von 90 - 220°C liegt, beispielsweise bei 15 - 90°C oder 40 - 80°C. Die Reihenfolge, in der die vorgenannten Komponenten zusammengegeben werden, ist beliebig. Bewährt hat es sich zum Beispiel, in eine 50 - 80°C warme Schmelze des Phenols zunächst den Katalysator, dann den Formaldehyd und schließlich das Mercaptan zu geben und das Gemisch anschließend auf die erfindungsgemäßen Temperaturen zu bringen. Es ist aber auch ohne Nachteil möglich, die Reihenfolge der Zugabe von Katalyator, Formaldehyd und Mercaptan zu vertauschen. Selbstverständlich kann man auch zwei, drei oder alle vier Komponenten simultan vermischen. Auch ist es möglich, den Formaldehyd und das Mercaptan in Form des betreffenden Thiomethanols (Halbmercaptals) zum Phenol/Katalysator-Gemisch einzudosieren oder auch eine umgekehrte Reihenfolge zu wählen. Natürlich ist es auch möglich, die Vermischung der Komponenten bei einer der erfindungsgemäßen Temperaturen durchzuführen, wobei allerdings dann den Dosierzeiten u.U. mehr Aufmerksamkeit gewidmet werden muß. Wird der Formaldehyd vor dem Mercaptan zum Phenol/Katalysator-Gemisch gegeben, so sollte die Mercaptan-Zugabe im allgemeinen um so schneller nachfolgen, je höher die Temperatur des Gemisches ist.

Die Isolierung des entstandenen 2-Alkyl- bzw. 2-Arylthiomethylphenols erfolgt durch Abtrennen des nicht umgesetzten bzw. des überschüssigen Phenols und des gegebenenfalls mitverwendeten Verdünnungsmittels nach bekannten Methoden. Das nichtumgesetzte bzw. überschüssige Phenol kann bei der erfindungsgemäßen Umsetzung wiederverwendet werden, ebenso das gegebenenfalls mit verwendete Verdünnungsmittel. Nebenprodukte sind gegebenenfalls ganz oder teilweise nach gängigen Verfahren zu entfernen. Das als Nebenprodukt anfallende Wasser kann auch schon während der Reaktion ganz oder teilweise entfernt werden.

Die nach dem erfindungsgemäßen Verfahren herstellbaren Alkyl- bzw. Arylthiomethylphenole können als Zwischenprodukte zur Synthese von Pflanzenschutzmitteln, insbesondere insektiziden Wirkstoffen wie z.B. 2-(Ethylthiomethylphenyl)-N-methyl-carbamat, verwendet werden (vgl. DT-PS 1 910 588, DE-OS 2 122 311).

Beispiel 1

Zu 564 g (6 Mol) aufgeschmolzenem Phenol von etwa 75°C in einem mit Innentemperatur, Rückflußkühler und Rührer ausgerüsteten Kolben werden 0,85 g Zinkacetat (≙ 0,00465 Mol $Zn^{++}$) in Form einer ca. 20 %igen wäßrigen Lösung gegeben, dann 30 g Paraformaldehyd mit 4 % Wassergehalt (≙ 0,96 Mol $CH_2O$) und schließlich 62 g ca. 97 %iges Ethylmercaptan (≙ 0,97 Mol). Sodann wird das Gemisch innerhalb von 0,5 Stunden auf etwa 135°C erhitzt. Bei dieser Temperatur tritt Rückfluß ein. Nach insgesamt 6 Stunden bei 135° ± 5° wird abgekühlt. Für diesen Zeitpunkt ergibt sich jodometrisch ein Ethylmercaptan-Umsatz von 98 % und gaschromatographisch eine 2-Ethylthiomethylphenol-Ausbeute von 86 % d.Th., bezogen auf Formaldehyd, sowie ein Verhältnis von 2- zu 4-Ethylthiomethylphenol von 98,9 : 1,1. Bei der anschließenden schonenden Fraktionierung im Vakuum über eine Vigreuxkolonne destillieren nach dem Reaktionswasser und dem überschüssigen Phenol bei ≤2 mbar Kopfdruck 131,5 g 2-Ethylthiomethylphenol mit einer Reinheit von >98 % über.

Beispiel 2 (Vergleichsbeispiel)

Wird ein wei im Beispiel 1 angesetztes Gemisch aus 6 Mol Phenol, 0,00465 Mol Zinkacetat, 0,96 Mol Formaldehyd (als Paraformaldehyd) und 0,97 Mol Ethylmercaptan bei 75°C gerührt, so ergeben sich nach bestimmten Zeiten die in Tabelle 1 angeführten, gaschromatographisch bestimmten Ausbeuten an 2-Ethylthiomethylphenol (2-ETMP) und

2-Hydroxymethylphenol (2-HMP) sowie die jodometrisch bestimmten Ethylmercaptan-Umsätze:

Tabelle 1:

| | Ausbeute und Umsätze bei 75°C nach | | | | | |
|---|---|---|---|---|---|---|
| | 6 | 24 | 48 | 96 | 200 | Stunden |
| Ausbeute an | | | | | | |
| 2-HMP | 6 | 6 | 6 | 6 | 6 | % d.Th. |
| 2-ETMP | 1,5 | 3,5 | 6,5 | 12 | 23 | % d.Th. |
| Umsatz an Ethylmercaptan | 6 | 9 | 14 | 23 | 43 | % |

Der gleiche Versuch ohne Ethylmercaptan-Zusatz ergibt nach 6 Stunden bei 75°C eine 2-HMP-Ausbeute von 75 - 80 %.

Man sieht, daß einerseits die durch Zinkacetat katalysierte 2-HMP-Bildung durch den Ethylmercaptan-Zusatz praktisch zum Erliegen kommt, andererseits aber keine nennenswerte 2-ETMP-Menge gebildet wird, auch nicht bei sehr langen Reaktionszeiten. Der Ethylmercaptan-Umsatz ist etwa doppelt so hoch wie die ETMP-Bildung.

Bei den erfindungsgemäßen Temperaturen wird dagegen 2-ETMP in sehr kurzer Zeit (6 Stunden) sehr selektiv in hoher Ausbeute (86 % d.Th.) erhalten (vergleiche Beispiel 1).

Beispiel 3

Zu einer auf 130 - 135°C gehaltenen Lösung von 4 ml 20 %iger Zinkacetat-Lösung in 640 g (6 Mol) Phenol

werden 92 g eines nach der Vorschrift von T.G. LEVI, Gazz. chim. ital. 62 (1932), s. 777, erhaltenen Reaktionsproduktes aus Formaldehyd und Ethylmercaptan (Ethylthiomethanol) gegeben. Nach 6 Stunden bei 130 bis 135°C Innentemperatur ergibt die gaschromatographische Analyse des Reaktionsgemisches eine Ausbeute an 2-Ethylthiomethylphenol von 85 % d.Th. (bezogen auf Ethylthiomethanol) und ein Verhältnis von 2-/4-Ethylthiolmethylphenol von 98,5 : 1,5.

Beispiele 4 bis 26

Es wird wie in Beispiel 1 gearbeitet, jedoch mit verschiedenen Katalysatoren. Das Molverhältnis von Phenol : Formaldehyd : Ethylmercaptan beträgt 6 : 0,96 : 0,97. Als Formaldehyd wird Paraformaldehyd mit 4 % Wassergehalt verwendet. Die Katalysatormenge in Mol pro Mol Phenol ist der Tabelle 2 zu entnehmen ebenso wie die Reaktionstemperatur, die Reaktionszeit, der Ethylmercaptan-Umsatz, die auf Formaldehyd bezogene Ausbeute an 2-Ethylthiomethylphenol (2-ETMP) und das Verhältnis von 2-Ethylthiomethylphenol (2-ETMP) zu 4-Ethylthiomethylphenol (4-ETMP). Das Verhältnis von 2-/4-ETMP ist ein Maß für die ortho-Selektivität.

Die Beispiele 4 bis 6 sind Vergleichsbeispiele, die zeigen, daß bei den erfindungsgemäßen Temperaturen ohne Katalysator (Beispiel 4) oder mit nicht erfindungsgemäßen Katalysatoren die 2-ETMP-Ausbeuten geringer, vor allem aber die ortho-Selektivitäten wesentlich schlechter sind.

**Tabelle 2:** Einfluß verschiedener Katalysatoren auf die Phenol/Formaldehyd/Ethylmercaptan-Umsetzung zu 2-Ethylthiomethylphenol (2-ETHP)

| Beispiel-Nr. | Katalysator: Zugabeform | Katalysator: Mol/Mol Phenol | Reaktions-Temperatur [°C] | Reaktionszeit [Std] | Mercaptan-Umsatz [%] | 2-ETMP-Ausbeute (rel. $CH_2O$) [%/d. Theorie] | Verhältnis 2-ETMP | : | 4-ETMP |
|---|---|---|---|---|---|---|---|---|---|
| 4a) | ohne | - | 135-140 | 6 | 99 | 45 | 82 | : | 18 |
| 5a) | Triethylamin | $1,7.10^{-3}$ | 122-136 | 6 | 97 | 62 | 74 | : | 26 |
| 6a) | KOH | $2,8.10^{-3}$ | 116-131 | 6 | 95 | 68 | 82 | : | 18 |
| 7 | LiOH | $7,7.10^{-4}$ | 107-130 | 8 | 94 | 79 | 95 | : | 5 |
| 8 | $Mg(OCOCH_3)_2$ | $1.10^{-3}$ | 116-133 | 8 | 96 | 81 | 99 | : | 1 |
| 9 | $Ca(OCOCH_3)_2$ | $\sim 5.10^{-4}$ | 122-125 | 8 | 82 | 55 b) | 98 | : | 2 |
| 10 | $Sr(OH)_3$ | $7,6.10^{-4}$ | 105-128 | 8 | 91 | 52 b) | 97 | : | 3 |
| 11 | $Ba(OCOCH_3)_2$ | $\sim 7.10^{-4}$ | 131-133 | 7 | 96 | 70 | 96,7 | : | 3,3 |
| 12 | $Pb(OCOCH_3)_2$ | $7,6.10^{-4}$ | 128-135 | 6 | 98 | 85 | 98,7 | : | 1,3 |
| 13 | ZnO | $7,8.10^{-4}$ | 130-136 | 8 | 99 | 86 | 98,2 | : | 1,8 |
| 14 | $Cd(OCOCH_3)_2$ | $7,6.10^{-4}$ | 130-136 | 8 | 97 | 82 b) | 97,6 | : | 2,4 |
| 15 | $Cu(OCOCH_3)_2$ | $7,6.10^{-4}$ | 130-136 | 5 | 93 | 60 | 93 | : | 7 |
| 16 | $Fe_2O_3$ | $3,8.10^{-4}$ | 131-139 | 7 | 93 | 65 | 93 | : | 7 |
| 17 | $CO(OCOCH_3)_2$ | $7,6.10^{-4}$ | 131-136 | 6 | 97 | 75 | 97 | : | 3 |
| 18 | $Ni(OCOCH_3)_2$ | $7,6.10^{-4}$ | 133-137 | 6 | 99 | 56 | 92 | : | 8 |
| 19 | $Cr(OH)_3$ | $7,6.10^{-4}$ | 129-136 | 7 | 98 | 61 | 91 | : | 9 |
| 20 | $Mn(OCOCH_3)_2$ | $7,6.10^{-4}$ | 117-133 | 4 | 99 | 85 | 99 | : | 1 |
| 21 | Aluminiumphenolat | $6.10^{-3}$ | 124-125 | 8 | 86 | 55 b) | 96,4 | : | 4,6 |

0025519

Tabelle 2 (Fortsetzung)

| Bei-spiel-Nr. | Katalysator | | Reaktions-Temperatur [°C] | Reaktions-zeit [Std] | Mercaptan-Umsatz [%] | 2-ETMP-Ausbeute (rel. $CH_2O$) [%/d. Theorie | Verhältnis 2-ETMP : 4-ETMP |
|---|---|---|---|---|---|---|---|
| | Zugabeform | Mol/Mol Phenol | | | | | |
| 22 | $PbO_2$ | $6,4.10^{-4}$ | 123-134 | 7 | 94 | 76 | 97,5 : 2,5 |
| 23 | $Cu_2O$ | $1,2.10^{-3}$ | 130-138 | 7 | 98 | 60 | 93 : 7 |
| 24 | $FeSO_4$ | $7,2.10^{-4}$ | 132-140 | 6 | 98 | 61 | 93 : 7 |
| 25 | $MnO_2$ | $1,9.10^{-3}$ | 124-136 | 7 | 98 | 81 | 98,5 : 1,5 |
| 26 | $Al_2O_3$ | $7,5.10^{-4}$ | 132-138 | 6 | 98 | 61 | 97 : 3 |

a) Vergleichsbeispiele

b) Ausbeute kann bei längerer Reaktionszeit noch höher sein

c) Temperatur des Reaktionsgemisches unter Rückfluß bei Normaldruck

Beispiel 27 bis 32

In Tab. 3 sind die mit Zink bei verschiedenen Konzentrationsverhälnisse und Temperaturen erhaltenen Ausbeuten an 2-Ethylthiomethylphenol (2-ETMP), die Verhältnisse von 2-ETMP zu 4-ETMP sowie die Ethylmercaptan-Umsätze zusammengestellt. Die ETMP-Bestimmungen erfolgten - wie bei den übrigen Beispielen auch - gaschromatographisch, die Umsatzbestimmungen jodometrisch. Der Formaldehyd wurde als Paraformaldehyd mit 4 % $H_2O$-Gehalt eingesetzt, das Zink in Form einer 20 Gew.%-igen wäßrigen Zinkacetat-Lösung.

Beispiel 33

Zu 564 g (6 Mol) Phenol von etwa 70°C werden $4,5.10^{-3}$ Mol Zinkacetat in Form einer ca. 20 %igen wässrigen Lösung, 30 g 96 %iger Paraformaldehyd (≙ 0,96 Mol $CH_2O$) und 113,6 g 97 %iges Thiophenol (1 Mol) gegeben. Dann wird das Gemisch innerhalb von ca. 30. Minuten auf Rückflußtemperatur erhitzt und 7,5 Stunden unter diesen Bedingungen (Gemischtemperatur 154 bis 132°C) gehalten. Nach dieser Zeit ergibt die gaschromatographische Analyse eine Ausbeute an 2-Phenylthiomethylphenol von 81 % der Theorie (bezogen auf Formaldehyd) und ein Verhätnis von 2- zu 4- Phenylthiomethylphenol von über 99:1. Das bei einer anschließenden Destillation bei 140-150°C/0,6 - 0,9 Torr erhaltene 2-Phenylthiomethylphenol war 98 %ig.

Tab. 3: Einfluß der Konzentrationsverhältnisse und Temperaturen auf die 2-Ethylthiomethylphenol-Bildung

| Beispiel-Nr. | Zink-Konzentration Mol/Mol Phenol | Phenol Mol | $CH_2O$ Mol | $C_2H_5SH$ Mol | Temperatur °C | Zeit Std. | Mercaptan-Umsatz % | 2-ETMP-Ausbeute rel. $CH_2O$ % d. Th. | Verhältnisse 2-ETMP : 4-ETMP |
|---|---|---|---|---|---|---|---|---|---|
| 27 | $1,9.10^{-4}$ | 6 | 0,96 | 0,97 | 134-142[a)] | 8 | 98 | 90 | 98,5 : 1,5 |
| 28 | $1,5.10^{-3}$ | 6 | 0,96 | 0,97 | 127-135[a)] | 6 | 98 | 85 | 98 : 2 |
| 29 | $7,7.10^{-4}$ | 4 | 0,96 | 0,97 | 125-137[a)] | 7 | 98 | 76 | 98 : 2 |
| 30 | $7,7.10^{-4}$ | 10 | 0,96 | 0,97 | 140-143[a)] | 2,5 | 98 | 94 | 98,9 : 1,1 |
| 31 | $7,5.10^{-4}$ | 6 | 0,96 | 0,97 | 100 | 52 | 97 | 83 | 96,3 : 3,7 |
| 32 | $7,5.10^{-4}$ | 6 | 0,96 | 0,87 | 170[b)] | 1 | 95 | 87 | 97,5 : 2,5 |

a) Temperatur des Reaktionsgemisches unter Rückfluß

b) Unter Eigendruck im Stahlautoklaven

Patentansprüche

1. Verfahren zur Herstellung von 2-Alkyl- bzw. 2-Arylthiomethylphenolen der Formel

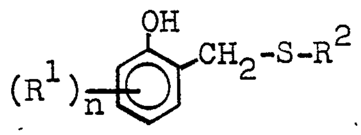

I

in der

n für 1, 2 oder 3 steht,

$R^1$ in 3-, 5- und/oder 6-Stellung zur Hydroxygruppe steht und entweder einzeln und unabhängig voneinander Wasserstoff, Alkyl, Cycloalkyl, Aryl, Aralkyl, Alkoxy- und/oder Halogen bedeutet,

$R^2$ für gegebenenfalls substituiertes Alkyl mit 1 bis 12 C-Atomen, für gegebenenfalls substituiertes Cycloalkyl mit 3 bis 6 C-Atomen, für gegebenenfalls substituiertes Phenyl oder gegebenenfalls substituiertes Aralkyl steht,

durch gleichzeitiges Umsetzen von Phenolen der Formel

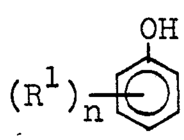

II

mit Formaldehyd oder einer Formaldehyd liefernden Verbindung und einem Mercaptan oder Thiophenol der Formel

$$R^2\text{-S-H} \qquad \text{III}$$

dadurch gekennzeichnet, daß man die Umsetzung bei Temperaturen von 90 bis 220°C in Gegenwart von Lithium-, Magnesium-, Calcium-, Strontium-, Barium-, Zink-, Cadmium-, Blei-, Mangan-, Kobalt-, Nickel-, Kupfer-, Eisen-, Chrom-, und/oder Aluminium-Verbindungen als Katalysator durchführt.

2. Verfahren nach Anspruch 1, dadurch gekennzeichnet, daß man die Umsetzung in Gegenwart von 0,1 bis $5.10^{-6}$ Mol Katalysatorverbindung pro Mol Phenol durchführt.

3. Verfahren nach Anspruch 1, dadurch gekennzeichnet, daß man die Umsetzung in Gegenwart von $1.10^{-2}$ bis $1.10^{-5}$ Mol Katalysatorverbindung pro Mol Phenol durchführt.

4. Verfahren nach Anspruch 1, dadurch gekennzeichnet, daß man Lithium-, Magnesium-, Calcium-, Strontium-, Barium-, Zink-, Cadmium-, Mangan-, Blei- und/oder Cobalt-Verbindungen als Katalysator verwendet.

5. Verfahren nach Anspruch 1, dadurch gekennzeichnet, daß im Phenol der Formel (II) $n = 1$ ist.

6. Verfahren nach Anspruch 1, dadurch gekennzeichnet, daß im Phenol der Formel (II) $n = 1$ und $R^1 = H$ ist.

0025519

Europäisches Patentamt

## EUROPÄISCHER RECHERCHENBERICHT

Nummer der Anmeldung

EP 80104895.0

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| | AT - B - 348 982 (BAYER)<br>+ Patentanspruch 1 +<br>-- | 1 | C 07 C 149/36<br>C 07 C 148/00 |
| | US - A - 3 553 270 (JOHN C. WOLLENSAK)<br>+ Patentanspruch 1 +<br>-- | 1 | |
| | US - A - 3 260 758 (FRANCIS X. O'SHEA)<br>+ Patentanspruch 1 +<br>-- | 1 | RECHERCHIERTE SACHGEBIETE (Int. Cl.3) |
| | US - A - 3 260 757 (FRANCIS X. O'SHEA)<br>+ Patentanspruch 1 +<br>-- | 1 | C 07 C 149/00<br>C 07 C 148/00 |
| | DE - B - 1 643 559 (UNIROYAL)<br>+ Spalte 1, Zeilen 1-17; Spalte 1, Zeile 56 - Spalte 2, Zeile 61 +<br>-- | 1 | |
| | GB - A - 1 499 043 (TEXACO)<br>+ Patentansprüche 1-3, 5, 13-16 +<br>---- | 1,2,4 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| WIEN | 14-11-1980 | REIF |